(19) Europäisches Patentamt
European Patent Office
Office européen des brevets

(11) **EP 1 226 227 B1**

(12) **EUROPÄISCHE PATENTSCHRIFT**

(45) Veröffentlichungstag und Bekanntmachung des
Hinweises auf die Patenterteilung:
**20.09.2006 Patentblatt 2006/38**

(51) Int Cl.:
*C12M 1/12* (2006.01) *C12M 3/06* (2006.01)

(21) Anmeldenummer: **00975788.1**

(22) Anmeldetag: **21.09.2000**

(86) Internationale Anmeldenummer:
**PCT/DE2000/003305**

(87) Internationale Veröffentlichungsnummer:
**WO 2001/021759 (29.03.2001 Gazette 2001/13)**

(54) **VERFAHREN ZUR KULTIVIERUNG VON ZELLEN, MEMBRANMODUL, VERWENDUNG EINES MEMBRANMODULS UND REAKTIONSSYSTEM ZUR KULTIVIERUNG VON ZELLEN**

METHOD FOR CULTIVATING CELLS, A MEMBRANE MODULE, UTILIZATION OF A MEMBRANE MODULE AND REACTION SYSTEM FOR CULTIVATION OF SAID CELLS

PROCEDE DE CULTURE DE CELLULES, MODULE MEMBRANAIRE, UTILISATION D'UN MODULE MEMBRANAIRE ET SYSTEME DE REACTION POUR LA CULTURE DE CELLULES

(84) Benannte Vertragsstaaten:
**AT BE CH CY DE DK ES FI FR GB GR IE IT LI LU MC NL PT SE**
Benannte Erstreckungsstaaten:
**LT LV MK RO SI**

(30) Priorität: **21.09.1999 DE 19945162**

(43) Veröffentlichungstag der Anmeldung:
**31.07.2002 Patentblatt 2002/31**

(73) Patentinhaber: **Aventis Pharma Deutschland GmbH**
**65929 Frankfurt am Main (DE)**

(72) Erfinder:
• **MÄRKL, Herbert**
**D-21218 Seevetal (DE)**
• **MAHR, Klaus**
**65239 Hochheim (DE)**
• **EISBRENNER, Günther**
**60529 Frankfurt am Main (DE)**
• **STAHL, Reiner**
**88400 Biberach (DE)**

(56) Entgegenhaltungen:
EP-A- 0 371 783     WO-A-00/46354
WO-A-87/06610     US-A- 3 915 802
US-A- 4 806 484

• **PATENT ABSTRACTS OF JAPAN vol. 015, no. 413 (C-0877), 22. Oktober 1991 (1991-10-22) & JP 03 172170 A (ASAHI MEDICAL CO LTD), 25. Juli 1991 (1991-07-25)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Kultivierung von Zellen unter Verwendung eines Reaktionssystems, wobei das Reaktionssystem einen Behälter für Dialyseflüssigkeit, einen Raum für die Kultivierung von Zellen sowie ein beide Räume miteinander verbindendes Membranmodul umfaßt, wobei das Membranmodul mindestens zwei durch eine Membran getrennte Räume aufweist, deren einer von einer Dialyseflüssigkeit und deren anderer von Zellen enthaltender Kulturflüssigkeit durchströmt wird, und ein erstes Gas in die Kulturflüssigkeit in dem Raum für die Kultivierung der Zellen eingetragen wird. Weiterhin betrifft die Erfindung ein Membranmodul, die Verwendung eines Membranmoduls und ein Reaktionssystem zur Kultivierung von Zellen.

Der Einsatz von Dialysemembranen im Zusammenhang mit der Kultur von Mikroorganismen bzw. von tierischen Zellen hat gewisse Vorteile in Bezug auf die erreichbaren Zelldichten bzw. der Konzentration der erhaltenen Produkte. Eine Zusammenfassung unterschiedlicher biotechnischer Produktionsverfahren, bei denen Membranen eingesetzt werden, wird von R. Pörtner und H. Märkl (Appl. Microbiol. Biotechnol. (1998) 50:403-414) gegeben. Der wesentliche Effekt der Verwendung von Dialysemembranen beruht darauf, daß wachstumshemmende Stoffwechselprodukte der eingesetzten Organismen, die während des Wachstums bzw. während der Produktion entstehen, über eine Membran abgezogen werden. Der Transport wird hierbei durch eine Konzentrationsdifferenz der genannten Stoffwechselprodukte angetrieben. In der Europäischen Patentschrift EP 0 632 827 B1 sind ein Verfahren und eine Vorrichtung beschrieben, in der die technische Nutzbarmachung des Konzepts erläutert wird. Genauer gesagt wird ein Reaktionssystem beschrieben, das aus mindestens zwei Kammern besteht, die über mindestens eine Membran miteinander gekoppelt sind und bei dem die Kultur von Mikroorganismen oder pflanzlichen oder tierischen Zellen sich in der einen Kammer befindet und die wachstumshemmenden Stoffwechselprodukte aus dieser Kammer in die Lösung der anderen Kammer gelangen.

Der dort beschriebene Einbau einer Dialysemembran direkt in den für die Kultur der Organismen vorgesehenen Raum ist im Labormaßstab (2-1-Maßstab) technisch gelöst (vergl. auch R. Pörtner und H. Märkl, aaO.). Das Scale-up dieser Technik in den Produktionsmaßstab bis zu mehreren m$^3$ beinhaltet jedoch Schwierigkeiten, die bis heute noch nicht beherrscht werden. Der Kern des Problems kann einfach erläutert werden. Es werden Membranen mit möglichst geringen Wandstärken (von wenigen $\mu$m bis zu 100 $\mu$m) angestrebt, da diese eine hohe Dialyseleistung besitzen. Diese Membranen verfügen jedoch nur über eine begrenzte mechanische Festigkeit. Die mechanische Belastung im Kulturraum ist jedoch bei schnell wachsenden aeroben Organismen sehr hoch, da es notwendig ist, große Mengen von Luft bzw. Sauerstoff für die Versorgung der Organismen einzutragen. Gleichzeitig müssen große Mengen von Kohlendioxid abtransportiert werden. Dieses bedingt einen hohen Energieeintrag durch Rühren in der Größenordnung von 10 kW/m$^3$, wodurch die dünnen Membranen stark belastet werden.

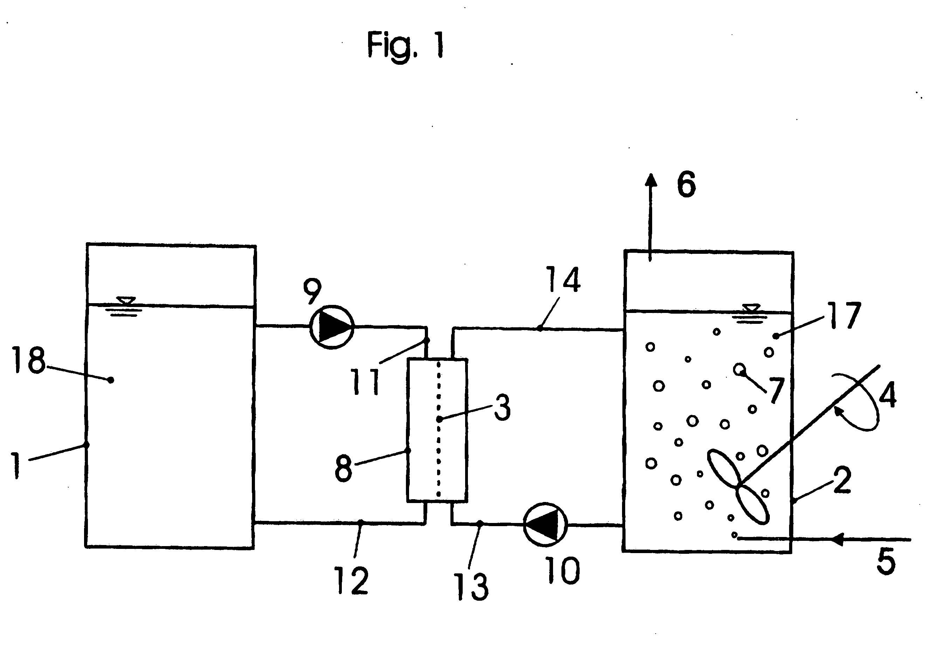

In dieser Hinsicht wesentlich unproblematischer ist eine andere, ebenfalls bekannte Anordnung, wie sie in Fig. 1 dargestellt ist. Hier ist die Dialysemembran 3 in einem eigenen Membranmodul 8 außerhalb des Kulturraumes 2 untergebracht. Die Kulturflüssigkeit wird mit Hilfe einer Pumpe 10 durch das Membranmodul 8 gepumpt. Entsprechend wird das Membranmodul 8 über eine zweite Pumpe 9 mit Dialyseflüssigkeit aus dem Behälter 1 versorgt. Das Scale-up dieser Anordnung in den großen Produktionsmaßstab ist unproblematisch, da die Anordnung im wesentlichen aus drei voneinander unabhängigen Einheiten, zwei Behälter und einem Membranmodul, besteht, die unabhängig von einander dimensioniert werden können. Diese Anordnung hat jedoch, wie von Ogbonna, J. Ch. und Märkl, H.. (Biotechnology and Bioengineering, Vol. 41, S. 1092-1100 (1993)), am Beispiel dichter E.coli-Kulturen gezeigt wurde, den erheblichen Nachteil, daß ein Teil der Mikroorganismen für eine gewisse Zeit den Kulturraum 2 verläßt und während der Dialyse im Membranmodul 8 nicht mit Sauerstoff versorgt wird. Im zitierten Beispiel wird die Zeit, die ein bestimmter Organismus auf seinem Weg durch das Dialysemodul außerhalb des begasten Kulturraumes verbringt, mit 11 Sekunden angegeben. Als Folge dieser sich wiederholenden zeitlich begrenzten Unterversorgung verringert sich die erreichbare Zelldichte auf einen Wert von 110 g Trockengewicht/Liter. In einer allerdings nicht Scale-up-fähigen Anordnung mit in den Kulturraum integrierter Membran, also bei zeitlich ununterbrochener Sauerstoffversorgung, wird unter sonst gleichen Bedingungen eine Organismendichte von 160 g Trockengewicht/Liter erreicht.

Der Erfindung liegt die Aufgabe zugrunde, das im Stand der Technik bekannte Reaktionssystem für die Kultivierung von Zellen nach Fig. 1, das mindestens einen Raum für die Kultiverung von Zellen (2), einen Behälter für Dialyseflüssigkeit (1)und ein dazwischengeschaltetes Membranmodul (8), durch das wachstumshemmende Stoffe aus der Kulturflüssigkeit in die Dialyseflüssigleit gelangen, umfaßt, so weiter zu entwickeln, daß höhere Zelldichten erreicht werden.

Eine weitere Aufgabe bestand in der Bereitstellung eines zur Durchführung des erfindungsgemäßen Verfahren geeigneten Reaktionssystems.

Erfindungsgemäß wird die Aufgabe gelöst durch ein Verfahren zur Kultivierung von Zellen unter Verwendung eines Reaktionssystems, wobei das Reaktionssystem einen Behälter für Dialyseflüssigkeit, einen Raum für die Kultivierung von Zellen sowie ein beide Räume miteinander verbindendes Membranmodul umfaßt, wobei das Membranmodul mindestens zwei durch eine Membran getrennte Räume aufweist, deren einer von einer Dialyseflüssigkeit und deren anderer von Zellen enthaltender Kulturflüssigkeit durchströmt wird, und ein erstes Gas in die Kulturflüssigkeit in dem Raum für die Kultivierung der Zellen eingetragen wird und ein zweites Gas in die Kulturflüssigkeit im Membranmodul eingetragen wird.

Insbesondere wird die Aufgabe gelöst durch ein Verfahren zur Kultivierung von Zellen unter Verwendung eines Reaktionssystems, wobei das Reaktionssystem einen Behälter für Dialyseflüssigkeit, einen Raum für die Kultivierung von Zellen sowie ein beide Räume miteinander verbindendes Membranmodul umfaßt, wobei das Membranmodul mindestens zwei durch eine Membran getrennte Räume aufweist, deren einer von einer Dialyseflüssigkeit und deren anderer von Zellen enthaltender Kulturflüssigkeit durchströmt wird, ein erstes Gas in die Kulturflüssigkeit in dem Raum für die Kultivierung der Zellen eingetragen wird und ein zweites Gas in die Kulturflüssigkeit im Membranmodul eingetragen wird, und die Membran funktional eine Dialysemembran ist.

In einer Ausführungsform des erfindungsgemäßen Verfahrens ist vorgesehen, daß eine direkte Begasung der im Membranmodul befindlichen Kulturflüssigkeit erfolgt, indem das zweite Gas direkt in die Kulturflüssigkeit im Membranmodul eingetragen wird.

In einer alternativen Ausführungsform des erfindungsgemäßen Verfahrens ist vorgesehen, daß eine indirekte Begasung der im Membranmodul befindlichen Kulturflüssigkeit erfolgt, indem das zweite Gas in die Dialyseflüssigkeit in dem Behälter für die Dialyseflüssigkeit eingetragen wird und von dort über die Membran des Membranmoduls in die im Membranmodul befindliche Kulturflüssigkeit gelangt.

In einer bevorzugten Ausführungsform der erfindungsgemäßen Verfahren erfolgt neben der direkten Begasung gleichzeitig die indirekte Begasung.

In einer Ausführungsform umfaßt das Membranmodul mindestens ein Gaszufuhrmittel und führt das Gaszufuhrmittel das zweite Gas zu mindestens einem der durch die Membran getrennten, eine Flüssigkeit führenden Räume zu.

Dabei ist bevorzugt, daß das Gaszufuhrmittel eine Austrittsöffnung aufweist, die sich insbesondere in dem die Kulturflüssigkeit führenden Raum des Membranmoduls befindet.

In einer besonders bevorzugten Ausführungsform ist das Gaszufuhrmittel ein Röhrchen.

In einer ganz besonders bevorzugten Ausführungsform ist das Gaszufuhrmittel als Düse ausgebildet.

In einer Ausführungsform ist vorgesehen, daß die Membran des Membranmoduls aus einem Material besteht, das ausgewählt ist aus der Gruppe, die regenerierte Cellulose, Polyamid, Polypropylen und Polysulfon umfaßt.

In einer weiteren Ausführungsform ist vorgesehen, daß das Membranmodul ein Plattenmodul ist.

In einer Ausführungsform des erfindungsgemäßen Verfahrens wie auch des erfindungsgemäßen Membranmoduls ist vorgesehen, daß die Membran des Membranmoduls eine Dialysemembran ist.

Dabei ist besonders bevorzugt, daß das Material der Dialysemembran Cuprophan ist.

In einer bevorzugten Ausführungsform der erfindungsgemäßen Verfahren sieht das Membranmodul bedingt durch sein Flächen-Volumen-Verhältnis und/oder seinen Permeabilitätskoeffizienten für das Gas einen für die Zellen ausreichenden Gasaustausch vor.

In einer Ausführungsform ist dabei vorgesehen, daß das Flächen-Volumen-Verhältnis mindestens etwa 5 $m^2$ pro Liter, insbesondere mindestens 10 $m^2$ pro Liter beträgt.

In einer bevorzugten Ausführungsform beträgt das Flächen-Volumen-Verhältnis etwa 13 $m^2$ pro Liter beträgt.

In einer weiteren Ausführungsform beträgt der Sauerstoff-Permeabilitätskoeffizient etwa 0,066 cm pro Minute oder mehr.

In eine Ausführungsform kann vorgesehen sein, daß der Behälter für Dialyseflüssigkeit ein Mittel zum Zuführen von Gas und zum Abführen von Gas aufweist.

Weiterhin kann bei den erfindungsgemäßen Verfahren vorgesehen sein, daß das Membranmodul, der Raum für die Kultivierung der Zellen und/oder der Behälter für Dialyseflüssigkeit einen erhöhten Druck aufweist.

Bei den erfindungsgemäßen Verfahren kann vorgesehen sein, daß das erste und zweite Gas einzeln und unabhängig voneinander ausgewählt ist aus der Gruppe, die Luft, Sauerstoff, Stickstoff, Kohlendioxid und Mischungen davon umfaßt.

In einer bevorzugten Ausführungsform ist das zweite Gas Sauerstoff.

In einer alternativen Ausführungsform ist das zweite Gas Kohlendioxid.

Bei den erfindungsgemäßen Verfahren kann vorgesehen sein, daß die Zellen ausgewählt sind aus der Gruppe, die mikrobielle Zellen, Pilzzellen, tierische Zellen und pflanzliche Zellen umfaßt.

Ganz besonders bevorzugt ist, wenn die Zellen Zellen von Escherichia coli sind.

Weiterhin wird die Aufgabe gelöst durch ein Membranmodul welches mindestens zwei durch eine Dialysemembran getrennte Räume umfaßt und jeder Raum mit einer Flüssigkeit durchströmt wird, die Flüssigkeit in einem Raum Dialyseflüssigkeit und in dem anderen Raum Kulturflüssigkeit ist, und wobei das Membranmodul mindestens ein Gaszufuhrmittel umfaßt und das Gaszufuhrmittel in mindestens einem der durch die Dialysemembran getrennten Räume eine Austrittsöffnung aufweist.

In einer Ausführungsform ist vorgesehen, daß die Membran röhrenförmig ausgebildet ist und durch ihr Volumen einen der beiden Räume ausbildet.

Dabei kann weiterhin vorgesehen sein, daß der Durchmesser des durch die röhrenförmige Membran ausgebildeten Raumes etwa 3 - 10 mm, bevorzugt 6 - 8 mm, beträgt.

In einer Ausführungsform umfaßt das erfindungsgemäße Membranmodul mehrere durch eine röhrenförmige Membran ausgebildete Räume.

In einer weiteren Ausführungsform befindet sich in mindestens einem der durch die röhrenförmige Membran ausgebildeten Räume eine Austrittsöffnung des Gaszufuhrmittels.

Dabei kann vorgesehen sein, daß sich die Austrittsöffnung des Gaszufuhrmittels in einem Raum außerhalb des/der durch die röhrenförmige Membran ausgebildeten Raumes/Räume befindet.

In einer bevorzugten Ausführungsform ist das Gaszufuhrmittel ein Röhrchen.

In einer ganz besonders bevorzugten Ausführungsform ist das Röhrchen konzentrisch in dem Raum angeordnet.

Bei dem erfindungsgemäßen Membranmodul kann vorgesehen sein, daß das Röhrchen einen Innendurchmesser von 0,2 mm bis 3 mm aufweist.

In einer besonders bevorzugten Ausführungsform ist die Austrittsöffnung des Gaszufuhrmittels als Düse ausgebildet.

Die Aufgabe wird erfindungsgemäß auch durch die Verwendung des erfindungsgemäßen Membranmoduls einem Verfahren nach der Erfindung gelöst.

Desweiteren wird die Aufgabe gelöst durch die Verwendung eines Membranmoduls, welches einen Gaspermeabilitätskoeffizienten, der hoch genug ist, um eine ausreichende Gasversorgung während der Passage der Kulturflüssigkeit durch das Membranmodul oder einen ausreichenden Gasaustausch in der im Membranmodul befindlichen Kulturflüssigkeit zu gewährleisten, und/oder ein geeignet hohes Flächen-Volumen-Verhältnis aufweist, um eine ausreichende Gasversorgung während der Passage der Kulturflüssigkeit durch das Membranmodul oder einen ausreichenden Gasaustausch in der im Membranmodul befindlichen Kulturflüssigkeit zu gewährleisten.

Genauere Angaben zu den beiden genannten Faktoren und wie diese zu bestimmen sind, ergeben sich aus der Beschreibung der Figuren und auch aus der Beschreibung der hierin offenbarten Verfahren.

Des weiteren wird die Aufgabe erfindungsgemäß gelöst durch ein Reaktionssystem zur Kultivierung von Zellen umfassend einen Behälter für Dialyseflüssigkeit, einen Raum für die Kultivierung von Zellen und mindestens ein dazwischen geschaltetes Membranmodul, wobei das Membranmodul ein erfindungsgemäßes Membranmodul ist.

In einer weiteren Ausführungsform enthält der Behälter für Dialyseflüssigkeit mindestens eine Begasungseinrichtung. Technische Voraussetzung dabei ist, dass der Behälter zumindest eine Leitung für die Zufuhr von Gas(en) enthält und mindestens eine (zweite) Leitung für den Abtransport von Gas(en).

In einer noch weiteren Ausführungsform weist die Membran des Membranmoduls einen Gaspermeabilitätskoeffizienten, der hoch genug ist, um eine ausreichende Gasversorgung während der Passage der Kulturflüssigkeit durch das Membranmodul oder einen ausreichenden Gasaustausch in der im Membranmodul befindlichen Kulturflüssigkeit zu gewährleisten und/oder ein geeignet hohes Flächen-Volumen-Verhältnis auf, um eine ausreichende Gasversorgung während der Passage der Kulturflüssigkeit durch das Membranmodul oder einen ausreichenden Gasaustausch in der im Membranmodul befindlichen Kulturflüssigkeit zu gewährleisten.

Der Erfindung liegt die überraschende Kenntnis zugrunde, daß durch Begasung der zu kultivierenden Zellen während der Passage durch das Membranmodul die zeitlich begrenzte Unterversorgung der Zellen mit Gas, insbesondere mit Sauerstoff im Falle von aeroben oder fakultativ aeroben Zellen, vermieden wird und somit höhere Zelldichten erreicht werden.

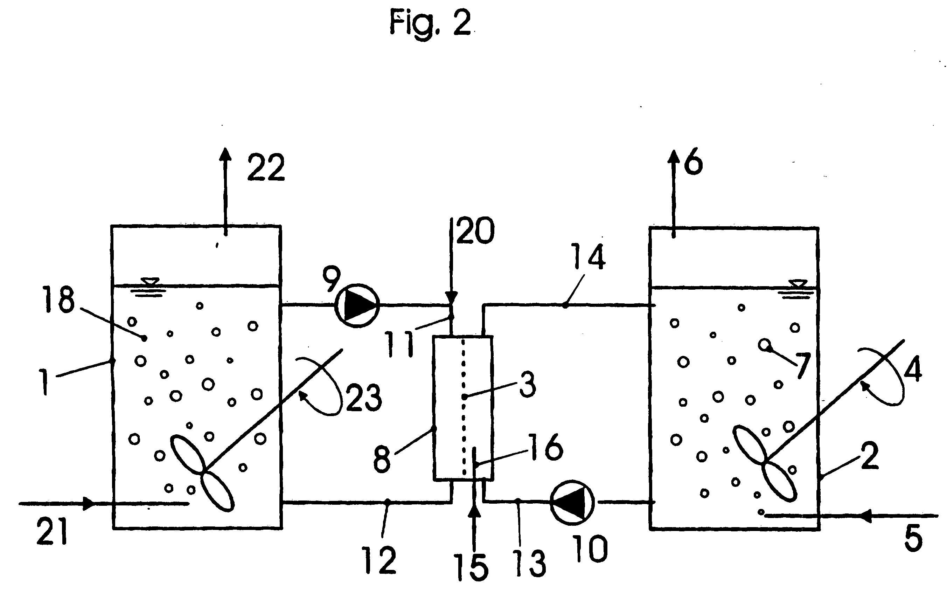

Der prinzipielle Aufbau des im Rahmen des erfindungsgemäßen Verfahrens verwendeten Reaktionssystems ist in Fig. 2 dargestellt. Obwohl in Fig. 2 nur ein Membranmodul dargestellt ist, kann das Reaktionssystem selbstverständlich mehrere parallel geschaltete Membranmoduleinheiten umfassen.

Im Rahmen des erfindungsgemäßen Verfahrens kann dabei vorgesehen sein, daß der erforderliche Gasaustausch oder die erforderliche Gaszufuhr, bspw. in Form der Zufuhr von Sauerstoff im Falle von aeroben oder fakultativ aeroben Organismen, direkt zu der im Membranmodul enthaltenen Kulturflüssigkeit erfolgt. Dies wird hierin als direkte Begasung bezeichnet. Die direkte Begasung kann dabei so erfolgen, daß Gas direkt über ein in das Membranmodul integriertes Gaszufuhrmittel (15,16) der - in der Regel Zellen enthaltenden - Kulturflüssigkeit zugeführt wird. Zu diesem Zweck kann im Rahmen der Erfindung das erfindungsgemäße Membranmodul verwendet werden bzw. dieses in dem Reaktionssystem, wie es im Rahmen des erfindungsgemäßen Verfahren verwendet wird, umfaßt sein.

Alternativ kann bei dem erfindungsgemäßen Verfahren der erforderliche Gasaustausch in der im Membranmodul enthaltenen Kulturflüssigkeit oder die dorthin erforderliche Gaszufuhr, wie sie hierin beschrieben ist, durch eine indirekte Begasung erfolgen. Die indirekte Begasung oder Gasversorgung erfolgt durch eine Begasung der Dialyseflüssigkeit 18 im Behälter für Dialyseflüssigkeit 1. Hierbei wird eine Gaszu- 21 und Gasabfuhrleitung 22 sowie in der Regel ein Rührwerk 23 eingesetzt. Die mit Gas angereicherte Dialyseflüssigkeit wird über eine Pumpe 9 durch das Membranmodul 8 gepumpt und kann über die Membran 3 das Gas an die Kulturflüssigkeit abgeben. Wichtige Parameter für die indirekte Begasung sind neben der Gaseintragsleistung in den Behälter mit Dialyseflüssigkeit der Permeabilitätskoeffizient der Membran des Membranmoduls für das jeweils betrachtete, d.h. eingetragene Gas und das Flächen-Volumen-Verhältnis des Membranmoduls.

Dabei ist es im Rahmen der Erfindung, daß bei dem erfindungsgemäßen Verfahren beide Formen der Begasung gleichzeitig angewandt werden, wobei es jedoch durchaus Phasen bei der Kultivierung der Zellen geben kann, wo auch nur eine der beiden Begasungsfomen angewandt wird.

Die ausschließlich direkte Begasung wie auch die ausschließliche indirekte Begasung stellen somit Extremfälle dar.

Je nach dem Verhältnis der für die Begasung zur Verfügung stehenden Membranfläche sowie deren Durchlässigkeit für das zu transportierende Gas und dem zu begasenden im Membranmodul 8 befindlichen Kulturvolumen, wird der Anteil der direkten (über die Gaszufuhrmittel 15,16) und der indirekten (über die Membran 3) Begasung unterschiedlich sein. Dabei ist grundsätzlich auch die in den Behälter mit Dialyseflüssigkeit, genauer die darin enthaltene Dialyseflüssigkeit, eintragbare Menge des betrachteten Gases zu berücksichtigen.

Ist beispielsweise durch die indirekte Begasung durch das Membranmodul 8 bedingt durch sein hohes Flächen-Volumen-Verhältnis und/oder den hohen Permeabilitätskoeffizienten der eingesetzten Membran für das in Frage stehende Gas, insbesondere Sauerstoff im Falle von aeroben und fakultativ aeroben Zellen, eine ausreichende Gasversorgung über die Membran möglich, kann die direkte Begasung über ein gesondertes Gaszufuhrmittel bzw. unter Verwendung des hierin offenbarten Membranmoduls entfallen.

Im allgemeinen kann die in den beschriebenen Membranmodulen eingesetzte Membran unabhängig vom Begasungstyp aus Cuprophan (regenerierte Zellulose), Polyamid, Polypropylen, Polysulfon oder anderen natürlichen oder synthetischen Stoffen bestehen. Es kann sich dabei um eine Dialysemembran (Ausschlußgröße ("cut off"): 10.000 Dalton), um eine mikroporöse Membran (Ausschlußgröße : 0,2 $\mu$m) oder um Membranen handeln, deren Durchlässigkeit (cut off) dazwischen liegt.

Es ist im Rahmen der vorliegenden Erfindung, dass die Membran auch im erfindungsgemäßen Membranmodul und/oder im erfindungsgemäßen Reaktionssystem funktional eine Dialysemembran ist.

Im Zusammenhang mit der erfindungsgemäßen Verfahren ebenso wie mit dem erfindungsgemäßen Membranmodul und Reaktionssystem haben alle Membranen den Charakter einer Dialysemembran.

Üblicherweise werden in der Technik Membranen aus regenerierter Zellulose (beispielsweise Cuprophan) als Dialysemembranen bezeichnet. Diese Membranen eignen sich nicht zum Einsatz als Filter, da diese (Dialyse-)Membranen hydraulisch nicht durchströmt werden. Die bei einer Dialysemembran wie einer Cuprophan-Membran bei transmembranen Druckunterschieden auftretenden hydraulischen Flüsse sind sehr gering. Die eigentliche Funktion dieser (Dialyse-) Membran beruht darauf, dass sich Konzentrationsunterschiede über die Membran durch Diffusion ausgleichen können.

Nimmt man nun beispielsweise eine mikroporöse Membran mit Poren kleiner 0,2 $\mu$m und vermeidet die Ausbildung einer transmembranen Druckdifferenz, so erfolgt auch in diesem Fall der Transport durch die Membran über die Mechanismen der Diffusion. Hydraulisches Durchströmen wird unter diesen Bedingungen weitgehend vermieden.

Im Rahmen des erfindungsgemäßen Verfahrens, das in der Regel ohne transmembrane Druckdifferenz durchgeführt wird, unterscheiden sich also poröse und insbesondere mikroporöse Membranen nicht von hydraulisch dichten Membranen (den eigentlichen Dialysemembranen).

Der hierin gewählte Begriff, dass die Membran funktional eine Dialysemembran ist, ist mit Blick auf diese Zusammenhänge zu verstehen. Mit anderen Worten, es kann im Rahmen des erfindungsgemäßen Verfahren bzw. des erfindungsgemäßen Membranmoduls bzw. des erfindungsgemäßen Reaktionssystems vorgesehen sein, dass eine Dialysemembran im eigentlichen Sinne verwendet wird. Es kann jedoch auch vorgesehen sein, dass eine poröse oder insbesondere mikroporöse Membran verwendet wird, sofern sich diese Membran funktional wie eine Dialysemembran verhält, d.h. keine hydraulische Durchströmung zeigt, oder, wenn eine hydraulische Durchströmung auftritt, deren Umfang relativ und insgesamt klein ist verglichen mit dem Stofftransport über die Membran durch Diffusion. Diese Änderung des Charakters der porösen bzw. mikroporösen Membran kann durch die Art, wie die Membran genutzt bzw. betrieben wird, bedingt werden, genauer dadurch, dass über die Membran kein Druck angelegt wird, wie er bspw. bei Perfusionssystemen anliegt.

Die vom Fachmann in diesem Zusammenhang anzustellenden Überlegungen werden in den Beispielen veranschaulicht.

Die Erfindung wird nun im folgenden anhand der beigefügten Zeichnungen weiter erläutert, wobei sich daraus weitere Vorteile und Ausführungsformen der Erfindung ergeben. Es zeigt

Fig. 1 ein Reaktionssystem nach dem Stand der Technik zur Kultivierung von Zellen umfassend einen Kulturraum 2, einen Behälter für Dialyseflüssigkeit 1 und ein eine Dialysemembran 3 enthaltendes separates Membranmodul 8;

Fig. 2 das erfindungsgemäße Reaktionssystem, das im Rahmen des erfindungsgemäßen Verfahrens verwendet wird,

Fig. 3 das erfindungsgemäße Membranmodul; und

Fig. 4 ein Plattenmodul der Firma Gambro Medizintechnik GmbH.

Fig. 1, die den grundlegenden Aufbau des gattungsgemäßen Reaktionssystems darstellt, wobei jene Version aufgezeigt wird, bei der das eine Dialysemembran 3 enthaltende Membranmodul 8 außerhalb des Kulturraumes 2 untergebracht ist, wurde bereits in der Einleitung der Beschreibung kurz diskutiert.

Kulturraum 2 enthält Kulturflüssigkeit 17, und im angeimpften Zustand die zu kultivierenden Zellen, die über Zuleitung 5 mit Sauerstoff versorgt werden. Die Sauerstoffversorgung kann dabei so erfolgen, daß Luft, Luft-Sauerstoff-Gemische oder reiner Sauerstoff zugeführt wird. Die an der Austrittsstelle der Zuleitung 5 gebildeten Gasbläschen 7 steigen in der Kulturflüssigkeit nach oben, wobei sie durch das Rührwerk 4 im Kulturraum 2 weiter dispergiert werden. Die verbleibenden Gase, ggf. gemeinsam mit den gasförmigen Reaktionsprodukten werden sodann über Ableitung 6 aus dem Kulturraum abgeleitet. Kulturraum 2 ist über Leitung 13 und 14 mit dem die Dialysemembran 3 enthaltenden Membranmodul 8 verbunden. Mittels Pumpe 10 wird Kulturflüssigkeit aus Kulturraum 2 in das Membranmodul gefördert und von dort über Leitung 14 wieder Kulturraum 2 zugeführt. Der Behälter für Dialyseflüssigkeit 1 ist mit Dialyseflüssigkeit gefüllt und über Leitung 11 und 12 mit dem Dialysemembran 3 enthaltenden Membranmodul 8 verbunden. Vermittels Pumpe 9 wird Dialyseflüssigkeit aus dem Behälter für Dialyseflüssigkeit 1 durch das Membranmodul 8 gefördert und über Leitung 12 dem Behälter für Dialyseflüssigkeit 1 wieder zugeführt.

Bei dieser schematischen Darstellung sind weitere Einrichtungen für das Reaktionssystem, wie sie den Fachleuten geläufig sind, nicht dargestellt, wie z. B. Meßeinrichtungen, Probenahmeeinrichtungen, Einrichtungen zur Medienzu- und -abfuhr sowie zur Substratzufuhr und -abfuhr, Einrichtungen zum Austausch der Dialyseflüssigkeit und dergleichen.

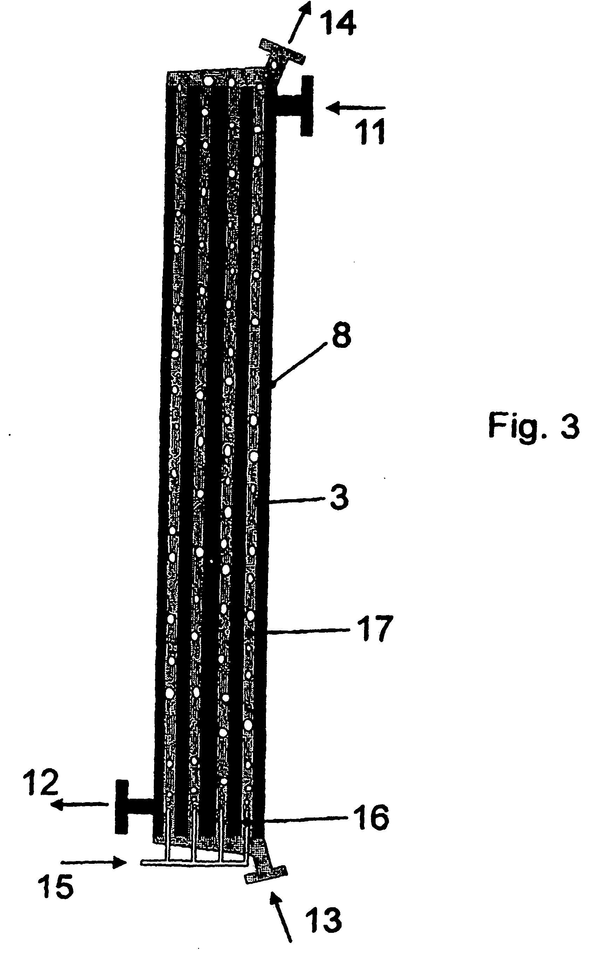

Bei dem erfindungsgemäßen Verfahren kann dabei das in Fig. 2 dargestellte erfindungsgemäße Reaktionssystem verwendet werden, welches eine Weiterentwicklung des in Fig. 1 gezeigten Reaktionssystems darstellt. Es umfaßt einen Behälter für Dialyseflüssigkeit, einen Raum für die Kultivierung der Zellen und ein dazwischengeschaltetes Membranmodul, welches als Dialysemodul fungiert. Das Membranmodul kann entweder das erfindungsgemäße Membranmodul, wie in einer Ausführungsform in Fig. 3 dargestellt, sein oder ein Membranmodul mit einem Gaspermeabilitätskoeffizienten, der hoch genug ist, um eine ausreichende indirekte Gasversorgung während der Passage der Kulturflüssigkeit durch das Membranmodul zu gewährleisten und/oder ein geeignet hohes Flächen-Volumen-Verhältnis aufweist, um eine ausreichende Gasversorgung während der Passage der Kulturflüssigkeit durch das Membranmodul zu gewährleisten, das erfindungsgemäß in dem erfindungsgemäßen Reaktionssystem verwendet wird. Es ist jedoch auch möglich, daß beide Begasungsarten gleichzeitig verwendet werden und sodann das Reaktionssystem wie in Fig. 2 dargestellt ausgestaltet ist.

Im folgenden werden die verschiedenen realisierbaren Begasungarten diskutiert. Sofern dabei Luft oder Sauerstoff als Gas angegeben wird, ist der jeweils angesprochene Aspekt nicht auf dieses spezielle Gas beschränkt, sondern dient lediglich der beispielhaften Veranschaulichung. Im Rahmen der vorliegenden Erfindung ist die Verwendung prinzipiell eines jeden Gases möglich.

Die direkte Begasung wird dabei so realisiert, daß im Membranmodul im unteren Bereich der röhrenförmigen Membranen Röhrchen 16 als Gaszufuhrmittel konzentrisch angeordnet werden, die in diesem Fall die Gaszufuhrmittel darstellen. Durch diese Röhrchen wird in der vorliegenden Ausführungsform unter erhöhtem Druck sauerstoffhaltiges Gas in den Membraninnenraum, d. h. den durch die röhrenförmige Membran ausgebildeten Raum, eingebracht, der Kulturflüssigkeit zusammen mit den zu kultivierenden Zellen enthält. Die Röhrchen sind im vorliegenden Fall untereinander durch eine Zuführleitung 15 verbunden. Die Röhrchen, deren Innendurchmesser 0,2 - 3 mm beträgt, können im Gasauslaßbereich eine Verengung enthalten, die als Düse ausgebildet sein kann, um die Menge des ausströmenden Gases zu begrenzen. Durch die Zuführleitung 15 wird der Organismenkultur Luft zugeführt, der auch Sauerstoff zugemischt werden kann. Es ist auch möglich, für die Begasung reinen Sauerstoff einzusetzen. Weiterhin ist es möglich, das Membranmodul insgesamt unter einen erhöhten Druck zu setzen, um den Partialdruck des Sauerstoffs im beschriebenen Gasgemisch zu erhöhen und damit die Sauerstoffzufuhr in die die Organismen enthaltende Suspension ebenfalls zu erhöhen.

Die indirekte Begasung erfolgt dabei in dem erfindungsgemäßen Verfahren dadurch, daß das Dialysegefäß 1 über eine Gaszufuhr 21 begast und damit die Dialyseflüssigkeit mit Sauerstoff angereichert wird. Das bezüglich Sauerstoff abgereicherte Gas verläßt den Dialysebehälter über die Leitung 22. Die sauerstoffhaltige Dialyseflüssigkeit wird über die Pumpe 9 in das Membranmodul 8 transportiert, wo der Sauerstoff über die Dialysemembran an die die zu kultivierenden Zellen, bspw. Mikroorganismen, enthaltende Suspension abgegeben wird. Die indirekte Gasversorgung, d.h. Sauerstoffversorgung hängt wesentlich vom Transportvermögen der Membran im vorliegenden Fall für Sauerstoff und/oder der verfügbare Membranfläche im Verhältnis zu dem zu versorgenden Volumen.

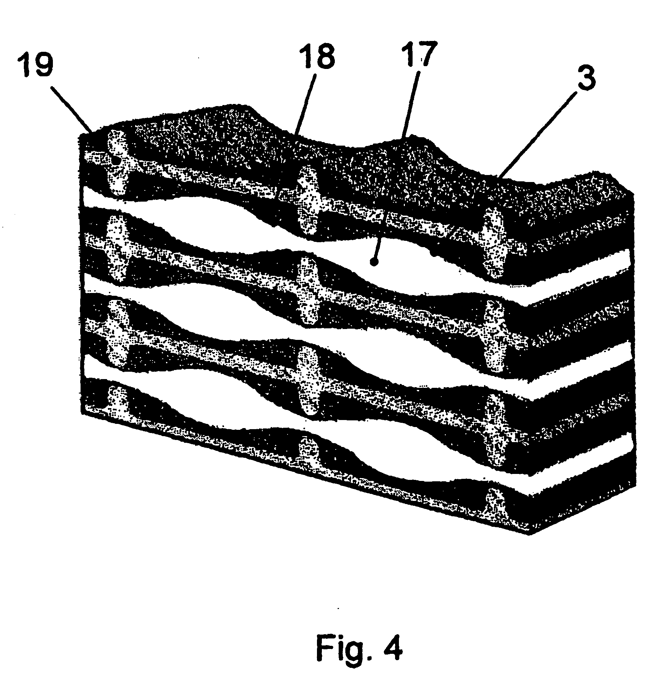

Diese Eigenschaften besitzt das Membranmodul der Firma Gambro, wie es in Fig. 4 schematisch dargestellt ist. Hierbei werden flache Membranen 3 in einem gewissen Abstand angeordnet, wobei jeweils zwei Membranen einen Raum ausbilden, der von der Kulturflüssigkeit 17 durchströmt wird. Der Außenbereich wird vom Dialysat 18 durchströmt. Die Membranen werden durch eine Stützstruktur 19 gehalten.

Eine detaillierte Beschreibung des Membranmoduls wird im Zusammenhang mit Fig. 4 gegeben.

Wie bereits ausgeführt kann im Rahmen des erfindungsgemäßen Verfahrens neben der direkten Begasung auch eine indirekte Begasung unter Verwendung eines wie in Fig. 4 gezeigten Membranmoduls erfolgen. Dabei kann auch vorgesehen sein, daß die indirekte Begasung über die Dialyseflüssigkeit entfallen kann, wenn die direkte Begasung ausreicht. Umgekehrt kann auch die direkte Begasung über das mit Gaszufuhrmitteln versehene Membranmodul, wie es in Fig. 3 beschrieben ist, entfallen, wenn das Membranmodul infolge seines hohen Flächen-Volumen-Verhältnisses und günstigen Transportverhaltens eine ausreichende Versorgung mit Gas gewährleistet.

Die Betriebsweise des aufgezeigten Reaktionssystems, seine apparatetechnische Ausstattung und das unter seiner Verwendung realisierbare Verfahren zur Kultivierung von Zellen entspricht ansonsten sinngemäß jener bzw. jenem, wie im Zusammenhang mit der erfindungsgemäßen Vorrichtung in Fig. 1 dargestellt. Dabei werden bevorzugterweise beide durch die Membran aufgespannten Räume mit dem annähernd gleichen hydrostatischen Druck beaufschlagt. Weiterhin ist bevorzugt, dass sich beide Behälter, deren Volumen mit der Membran in Verbindung stehen, auf annähernd gleichem Druckniveau befinden.

Tatsächlich haben Experimente mit der Kultur von *E.coli* und der in Fig. 2 angegebenen Anordnung unter Verwendung des in Fig. 4 dargestellten Membranmoduls und der dort diskutierten Sauerstoffversorgung in eigenen Experimenten hervorragende Ergebnisse erbracht. Die erreichten Zelldichten von 160 g Trockengewicht/Liter entsprechen jenen, die mit einem Reaktionssystem erreicht werden, bei dem die Membran direkt in den Reaktionsraum integriert war. Die Experimente zeigen, daß eine Beeinträchtigung des Wachstums durch zeitlich befristeten Sauerstoffmangel nicht auftritt.

Sollte in einem Fall eine weitere Erhöhung der Sauerstoffversorgung über die Membran gewünscht sein, so stehen hierfür weitere Maßnahmen zur Verfügung. Es ist beispielsweise möglich, durch eine Leitung 20 (Fig. 2) zusätzlich gasförmigen Sauerstoff in das Membranmodul auf Seiten der Dialyseflüssigkeit einzutragen. Dieser löst sich im Verlauf der Membranpassage in der Dialyseflüssigkeit. Somit erhöht sich das für die Sauerstoffversorgung maßgebliche Konzentrationsgefälle $(c_1-c_2)_{\text{Mittel}}$.

Eine Erhöhung der Sauerstoffkonzentration im Dialysat ist auch möglich, in dem das gesamte Druckniveau der Anordnung nach Fig. 2 angehoben wird. Bei einer Verdoppelung des Drucks vom Druckniveau der Umgebung von $10^5$ Pa auf $2 \cdot 10^5$ Pa, um ein Beispiel zu nennen, lassen sich auch die entsprechenden Sauerstoffgehalte im Dialysat verdoppeln. Damit erhöht sich entsprechend die Sauerstoffversorgung des Kulturmediums im Membran- bzw. Dialysemodul (Die Begriffe Membranmodul und Dialysemodul können hierin synonym verwendet werden.)

Bei dem erfindungsgemäßen Verfahren zum Kultivieren von Zellen sind ein oder mehrere der folgenden Schritte umfaßt, wobei die Kultivierung in einem derartigen gattungsgemäßen Reaktionssystem den Fachleuten auf dem Gebiet prinzipiell bekannt ist: a) Bereitstellen eines Reaktionssystems umfassend ein Kulturgefäß (Kulturraum), ein externes Membranmodul, welches die erfindungsgemäße Vorrichtung, d.h. das erfindungsgemäße Membranmodul oder ein kommerziell erhältliches Membranmodul mit den erforderlichen Eigenschaften (Flächen-Volumen-Verhältnis, Sauerstoffpermeabilitätskoeffizient) und einen Behälter für Dialyseflüssigkeit umfaßt, wobei das Reaktionssystem, wie auch die darin enthaltenen Flüssigkeiten, bevorzugt sterilisiert wurde, b) Inokulieren des Kulturgefäßes mit den zu kultivierenden Zellen, c) Bereitstellen einer geeigneten Temperatur, Substrat- und Sauerstoffversorgung, d) Führen der Kulturflüssigkeit, die die zu kultivierende Zellensuspension enthält, aus dem Kulturgefäß durch die Dialysevorrichtung und Rückführen der Kulturflüssigkeit zum Kulturgefäß unter gleichzeitigem Kreisführen der Dialyseflüssigkeit aus dem Behälter für Dialyseflüssigkeit durch die Dialysevorrichtung zurück in den Behälter für Dialyseflüssigkeit, wobei die beiden Flüssigkeitsströme bevorzugt im Gegenstrom geführt werden, und e) Ernten der kultivierten Zellen.

Obwohl die vorstehenden Ausführungen weitestgehend im Zusammenhang mit der Kultivierung von Mikroorganismen, insbesondere E.coli, erfolgt sind, ist die Verwendung der erfindungsgemäßen Vorrichtung, der Reaktionssysteme oder des Verfahrens in keinster Weise darauf beschränkt.

Bei den im Zusammenhang mit der Vorrichtung, der Verwendung, den Reaktionssystemen und den Verfahren der vorliegenden Erfindung verwendeten Zellen können auch photosynthetische Zellen verwendet werden, unabhängig davon, ob es sich dabei um prokaryontische oder eukaryontische Zellen, mithin um photosynthetisch aktive mikrobielle oder pflanzliche Zellen handelt.

Ein Aspekt der vorliegenden Erfindung betrifft somit die Versorgung von Zellen mit Sauerstoff.

Ein anderer Aspekt der vorliegenden Erfindung betrifft die Beseitigung von Gasen bzw. gasförmigen Stoffwechselprodukten, wie sie in Kulturen von Zellen auftreten. Dabei spielt die Überlegung eine Rolle, daß der Vorgang des Eintragens von Gasen im Prinzip, zumindest was die physiko-chemischen Vorgänge dabei anbelangt, ähnlich dem des Austragens von Gasen ist. Dabei kann unter dem Einfluß des Einführens, insbesondere Einblasens, eines Gases, ein anderes oder gleiches Gas aus der Flüssigkeit entfernt werden. Im vorliegenden Fall kann dieses Flüssigkeit die Kulturraumflüssigkeit (Medium) und/oder die Dialyseflüssigkeit sein. Insoweit kann die erfindungsgemäße Vorrichtung dazu verwendet werden, Gase aus der Flüssigkeit in dem/den Reaktionssystem(en) oder einem Teil davon zu entfernen.

Dies gilt auch für den Fall der erfindungsgemäßen der Verwendung eines Membranmoduls.

Beispielhaft seien einige derartige Kultivierungssysteme angeführt, bei denen sich das Strippen gasförmiger Stoffwechselprodukte als vorteilhaft erwiesen hat.

Bei photosynthetisch aktiven Zellen, beispielsweise pflanzlichen Zellen oder Algen, wird typischerweise Kohlendioxid dem Kulturmedium zugesetzt. Dies kann unter Verwendung eines entsprechend ausgelegten Membranmoduls oder der erfindungsgemäßen Vorrichtung erfolgen, wobei für die Auslegung der Begasungsvorrichtung bzw. des verwendeten membranmoduls Berechnungen anzustellen sind, die ähnlich den obigen zur Sauerstoffversorgung von Escherichia coli-Kulturen sind, und den Fachmann in die Lage versetzen, die erforderliche Menge an Kohlendioxid der Kultur zuzuführen. Gleichzeitig ist es bei hochdichten Kulturen derartiger Zellen erforderlich, den infolge der photosynthetischen Aktivität der Zellen entstandenen Sauerstoff aus dem Medium zu entfernen. Auch dies kann durch die erfindungsgemäße Vorrichtung bzw. Verwendung eines Membranmoduls bewerkstelligt werden. Die Begasung wird, unter Einstellung geeigneter Partialdrücke, typischerweise unter Verwendung eines Gemisches aus Luft und Kohlendioxid erfolgen.

Ein weiteres Beispiel für den Aspekt der Erfindung betreffend das Entfernen von Gasen aus Flüssigkeiten gemäß der vorliegenden Erfindung stellt die Kultivierung von Pyrococcus dar. Hier ist die Begasung mit Stickstoff, oder einem Gemisch aus Stickstoff und Kohlendioxid für das Erreichen hoher Zelldichten erfolgreich. Die entsprechende Offenbarung hierzu von Krahe, M. FEMS Microbiology reviews 18 (1995) 271 - 285 wird hierin durch Bezugnahme aufgenommen.

Der Fachmann wird somit, wann immer die Gaszufuhr bzw. -abfuhr für die Kultivierung von Zellen hilfreich oder erforderlich scheint, oder mit anderen Worten, der Gasstoffwechsel einer Kultur von Bedeutung für die Kultivierung der Zellen ist, auf die Vorrichtung, V-erwendung, Reaktionssysteme und Verfahren nach der vorliegenden Erfindung zurückgreifen (können).

Bei dem erfindungsgemäßen Verfahren kann das vorbeschriebene Reaktionssystem verwendet werden. Dabei kann vorgesehen sein, daß entweder das Behältnis 1 mit der Dialyseflüssigkeit oder das eigentliche Kulturgefäß, d. h. der Kulturraum, oder beide, batchweise, semikontinuierlich, oder kontinuierlich betrieben werden. Neben der Begasung in der erfindungsgemäßen Vorrichtung kann die Begasung im Kulturraum und/oder im Behälter für Dialyseflüssigkeit, genauer in der Dialyseflüssigkeit (oder Dialysat 18) erfolgen. Im Kulturgefäß können prinzipiell jegliche Formen von Zellen kultiviert werden, d. h. prokaryontische und eukaryontische Zellen, insbesondere Mikroorganismen, Pilzzellen, tierische Zellen und Pflanzenzellen. Im Kulturgefäß liegen die Zellen typischerweise in Suspension vor. Hinsichtlich der Betriebsweise, insbesondere des Kulturgefäßes, ist jede denkbare Fütterungsstragie möglich, so beispielsweise und insbesondere auch fedbatch oder split feed strategy, die beispielsweise beschrieben ist Ogbonna, J. Ch. und Märkl, H.; Biotechnology and Bioengineering Vol. 41, S. 1092 - 1100 (1993), deren Offenbarungsgehalt hierin vollumfänglich aufgenommen wird. Dabei wird entsprechend den Nährstoffbedingungen der Kultur selektiv die ansonsten limitierenden Substrate, wie beispielsweise eine Kohlenstoffquelle, eine Stickstoffquelle und/oder bestimmt Salze hinzugefügt.

Fig. 3 zeigt das erfindungsgemäße Membranmodul, das als Membranmodul 8 beispielsweise in das in Fig. 1 oder Fig. 2 gezeigte Reaktionssystem eingebaut werden kann, d. h. als separates Membranmodul 8 zwischen Kulturraum 2 und den Behälter für Dialyseflüssigkeit 1 zwischengeschaltet werden kann.

Der Aufbau des Membranmodules 8 besteht aus insgesamt zwei Gruppen von Räumen, die jeweils in einer Mehrzahl vorhanden sind, zum einen jene Räume, in denen sich Kulturflüssigkeit 17 befindet bzw. durch die diese strömt, und zum anderen jene Räume, die die Dialyseflüssigkeit enthalten. Im vorliegenden Fall bildet die Dialysemembran im Membranmodul 8 Röhren aus, hierin summarisch als röhrenförmige Membran bezeichnet, innerhalb derer die Kulturflüssigkeit geführt wird. Die dazwischen liegenden Räume, durch das Membranmaterial von der Kulturflüssigkeit getrennt, werden von der Dialyseflüssigkeit durchströmt. Das Modul ist über die Zuleitung 13 und die Ableitung 14 mit dem Kulturgefäß verbunden. Im Falle einer Suspensionskultur im Kulturraum 2 durchströmen die Zellen hierbei das Innere der röhrenförmigen Membran(en) von unten nach oben. Die Membranaußenwand wird von der Dialyseflüssigkeit umströmt, die über die Leitungen 11 und 12 mit dem Behälter für Dialyseflüssigkeit 1 ausgetauscht wird. Insoweit bilden Zuleitung 13 und Ableitung 14 und Zuleitung 11 und Ableitung 12 jeweils einen Satz für die Zu- und Abfuhr von flüssigem Medium zu und von dem durch die röhrenförmige(n) Membran(en) ausgebildeten Raum bzw. zu und von dem außerhalb des durch die röhrenförmige(n) Membran(en) ausgebildeten Raumes gelegenen Raum. In der vorliegenden Ausführungsform ist dabei vorgesehen, daß für die beiden Seiten des Dialysemoduls, d. h. Dialyseflüssigkeit bzw. zu dialysierende Flüssigkeit, d. h. Kulturflüssigkeit, nur jeweils eine Zu- bzw. Ableitung vorgesehen ist. Es ist jedoch auch möglich, jeweils mehrere dieser Zu- bzw. Ableitungen auf einer jeden Seite vorzusehen.

Sofern das erfindungsgemäße Membranmodul in dem erfindungsgemäßen Reaktionssystem eingebaut vorliegt, wird eine Druckerhöhung im Membranmodul zweckmäßigerweise durch eine gleichzeitige Erhöhung des Drucks im Behälter für Dialyseflüssigkeit 1 und dem Kulturraum 2 erreicht. Durch diese Maßnahme ist es möglich, wie eigene Experimente gezeigt haben, eine Sauerstoffversorgung im notwendigen Bereich zwischen 5 und 15 g/(1 h) [d.h. kg $O_2$ pro $m^3$ Kulturflüssigkeit und Stunde] ohne weiteres zu realisieren

Das Gas oder Gasgemisch strömt das durch die Zuführleitung 15 durch den Innenraum der Membran 3, verläßt das Membranmodul durch die Ableitung 14, gelangt von dort in das Kulturgefäß 2 und verläßt dieses zusammen mit dem Abgas des ebenfalls mit Sauerstoff versorgten Kulturraums 2 durch die Öffnung 6 im Deckel des Kulturraums 2.

Das Gas wird über die Röhrchen 16 in den Innenraum der Membran 3 unter einem gewissen Überdruck eingebracht. Abhängig von der Höhe des Überdruckes erhält das Gas eine gewisse Geschwindigkeit, mit der es in die die Mikroorganismen enthaltende Suspension eintritt. Da das Gas durch die Flüssigkeit abgebremst wird, überträgt sich ein Impuls auf die Flüssigkeit, die zur Förderung der Flüssigkeit im Inneren der röhrenförmigen Membran 3 beiträgt. Ein weiterer Fördereffekt entsteht aufgrund des hohen Gasgehalts, der sich im Membraninnenraum einstellt. Da dieser Gasgehalt bei entsprechend starker Gaszufuhr höher als im eigentlichen Kulturraum 2 ist, stellt sich ein zusätzlicher Pumpeffekt ein (Mammutpumpeneffekt). Aus diesem Grund kann bei entsprechend hoher Begasung des Membranmoduls auf die Förderpumpe 10 in einem die erfindungsgemäße Vorrichtung enthaltenden Reaktionssystem verzichtet werden.

Das Membranmodul 8 wird im Regelfall möglichst nahe am Kulturraum 2 angebracht, damit die unbegaste Zufuhrleitung 13 zwischen Kulturraum und Membranmodul möglichst kurz ist und demzufolge die in der Kulturflüssigkeit enthaltenen Zellen nur möglichst kurz den Bedingungen einer nicht optimalen Gasversorgung ausgesetzt sind.

Figur 4 zeigt einen Querschnitt durch ein Membranmodul, das erfindungsgemäß für das Begasen von Kulturflüssigkeit für die Kultivierung von Zellen verwendet werden kann. Der in Fig. 4 konkret dargestellte Querschnitt stammt von einem kommerziell erhältlichen Plattenmodul mit der Bezeichnung Gambro LunDia: Alpha 600 der Firma Gambro Medizintechnik GmbH. Das Membranmaterial ist Cuprophan®. Die Wandstärke der Membran beträgt 8 $\mu$m (im trockenen Zustand), die Membranfläche beträgt 1,3 m$^2$ und die Anzahl der Membranlagen pro Modul beträgt 70. Das Volumen des Raumes 17, der bei einem erfindungsgemäßen Betrieb Kulturflüssigkeit und Zellen enthält, beträgt 100 ml.

Derartige Membranmodule werden derzeit lediglich in der Hämodialyse verwendet und zu diesem Zweck hergestellt, nicht jedoch bei der Kultivierung von Zellen und insbesondere nicht in einem Reaktionssystem der gattungsgemäßen Art, wie dies schematisch für den Fall, daß das Membranmodul als eigenständige Einheit angeordnet ist, in Fig. 1 und Fig. 2 dargestellt ist.

Im Rahmen von Untersuchungen wurde überraschenderweise festgestellt, daß unter bestimmten Voraussetzungen auch diese kommerziell erhältlichen Membranmodule dann verwendet werden können, wenn infolge ihres Flächen-Volumen-Verhältnisses und des Permeabilitätskoeffizienten für Sauerstoff eine für die Zellen ausreichende indirekte Sauerstoffversorgung gewährleistet wird.

Hierzu sind einige im folgenden angeführte Überlegungen anzustellen und insbesondere die Permeabilitätskoeffizienten für Sauerstoff zu bestimmen, was jedoch im Rahmen der Fähigkeiten des Durchschnittsfachmannes erfolgen kann. Die Bestimmung von Permeabilitätskoeffizienten ist zum Beispiel für die Verbindung Glycerin beschrieben in Märkl, H. et al. Appl. Microbiol. Biotechnol. (1993) 39: 48-52.

Bei dem beispielhaft hier angesprochenen Plattenmodul Gambro LunDia Alpha 600 ermöglicht die vorgegebene Konstruktion ein extrem hohes Verhältnis von Membranfläche zu zu versorgendem Volumen.

Die aktive Membranfläche beträgt bei der Ausführung LunDia Alpha 600 1,3 m$^2$. Das Volumen des mit Sauerstoff zu versorgenden Raumes beträgt 100 ml, so daß das Flächen-Volumen-Verhältnis

$$F/V = 13 \text{ m}^2/\text{l (LunDia)}$$

beträgt.

Im Vergleich sei das entsprechende Flächen-Volumen-Verhältnis für ein Membranröhrchen angegeben, wie es in der in Fig. 2 skizzierten Anordnung eingesetzt wird. In diesem Fall muß der Innenraum des Röhrchens versorgt werden. Als Transportfläche steht die Mantelfläche der zylinderförmigen Membranfläche zur Verfügung. Am Beispiel eines Röhrchens mit 5 mm Durchmesser errechnet sich

$$F/V = 0,8 \text{ m}^2/\text{l (5 mm Röhrchen)}$$

Bei einem Röhrchendurchmesser von nur 1 mm erhält man einen Wert von

$$F/V = 4 \text{ m}^2/\text{l (1 mm Röhrchen).}$$

Eine Beispielrechnung zeigt, daß mit dem Membranmodul LunDia Alpha 600 tatsächlich über die Membran eine ausreichende indirekte Sauerstoffversorgung erreicht werden kann:

Der Sauerstofftransport, bezogen auf das zu versorgende Volumen, beträgt:

$$S = F/V * P * (c_1 - c_2)_{Mittel}$$

Hierbei ist $F/V = 13$ m$^2$/l.

P ist der Permeabilitätskoeffizient. Dieser wurde in eigenen Experimenten für eine Membran, Typ Cuprophan, wie sie im genannten Modul eingesetzt wird, zu $P = 0,066$ cm/min bestimmt. Dieser Wert gilt für eine Membran der Dicke 20 $\mu$m (Dicke gemessen im trockenen Zustand). Im Membranmodul LunDia Alpha 600 wird eine Cuprophan-Membran der Dicke 8 $\mu$m eingesetzt, so daß der tatsächliche Permeabilitätskoeffizient eher höher liegt und die vorgestellte Rechnung einen eher "unteren" Wert ergibt.

Das Konzentrationsgefälle $(c_1-c_2)$ über die Membran wird mit

$$(c_1-c_2)_{Mittel} = 11,45 \text{ mg/l}$$

angenommen. In diesem Rechenbeispiel wird davon ausgegangen, daß dem Membranmodul aus dem Behälter für Dialyseflüssigkeit 1 ein Dialysat mit einem Sauerstoffgehalt von 30 mg/l zufließt. Hierbei wird angenommen, daß der Behälter mit reinem Sauerstoff begast wird (die Sättigungskonzentration für reinen Sauerstoff beträgt ca. 32 mg/l). Im Membranmodul wird das Dialysat auf eine Konzentration von 4 mg/l abgereichert. Mit dieser Konzentration wird das Dialysat wieder dem Behälter zugeführt. Es ist also auf der Dialysatseite mit einem Sauerstoffgehalt $c_1$ zu rechnen, der sich von 30 mg/l (Moduleingang) auf 4 mg/l (Modulausgang) verringert. Der Sauerstoffgehalt auf der Kulturseite soll bei einem für das Wachstum der Organismen ausreichenden Wert von konstant $c_2 = 1$ mg/l liegen. Mit diesen Annahmen berechnet sich

$$(c_1 - c_2)_{Mittel} = \frac{(c_{1\,Eingang} - c_2) - (c_{1\,Ausgang} - c_2)}{\ln((c_{1\,Eingang} - c_2)/(c_{1\,Ausgang} - c_2))} = 11.45 \text{ mg/l}$$

Mit den angenommenen Daten ergibt sich ein Wert für die auf das Kulturvolumen bezogenen Sauerstoffversorgung von

$$S = 5,9 \text{ g/(l*h)}.$$

Da der tatsächliche Permeabilitätskoeffizient, der die Durchlässigkeit der Membran für Sauerstoff angibt, in der Realität wegen der geringen Membrandicke von nur 8 $\mu$m deutlich über dem in der Rechnung angenommenen Wert von 0,066 cm/min liegt, wird in der Realität eine bessere Sauerstoffversorgung der Organismensuspension in der Membran erreicht werden, als er hier rechnerisch angegeben wird. Es kann also davon ausgegangen werden, daß die Sauerstoffversorgung von Mikroorganismen in einem externen Membranmodul über die Membran, wenn das Flächen-Volumen-Verhältnis ausreichend groß ist und eine gute Permeabilität der Membran für Sauerstoff vorliegt, in einem Bereich liegt, der als "ausreichend" anzusehen ist.

Der Begriff "ausreichend" orientiert sich am Bedarf der jeweiligen Zellen bzw. an der Versorgung, wie sie in dem Kulturgefäß 2 realisiert wird. Bei der technischen Kultur von E. coli Zellen, um ein Beispiel zu nennen, sind Werte üblich, die im Bereich zwischen 5 und 15 g / (1 h) liegen.

Aufgrund dieser Zusammenhänge kann der Fachmann in Kenntnis bzw. nach Bestimmung des Sauerstoffpermeabilitätskoeffizienten sowie des Flächen-Volumen-Verhältnisses des jeweils verwendeten Moduls leicht ermitteln, ob dieses für die erfindungsgemäße Verwendung geeignet ist. Für den Fall, daß mit dem Membranmodul die erwünschte Versorgung mit Gas nicht gewährleistet werden kann, kann ein Membranmodul nach Fig. 3 verwendet werden und dann sowohl eine indirekte als auch direkte Sauerstoffversorgung vorgenommen werden.

Die vorstehend geschilderten Zusammenhänge wurde vom Erfinder völlig überraschend erkannt und insbesondere die Möglichkeit, das ansonsten lediglich für die Hämodialyse verwendete System in dem gattungsgemäßen Reaktionssystem als separates Membranmodul einzusetzen, wobei, wie im Zusammenhang mit Fig. 2 weiter erläutert ist, der Sauerstoffeintrag in die Dialyseflüssigkeit von Behälter 1 erfolgt und die solchermaßen angereicherte Dialyseflüssigkeit genügend Sauerstoff transportiert, um den Kulturraum 2 bzw. die darin enthaltenen Zellen zu versorgen und, in einem weiteren Aspekt, insbesondere die bei der Passage des Membranmoduls unter Sauerstofflimitierung leidenden Zellen in hinreichender bzw. erforderlicher Weise mit Sauerstoff zu versorgen, so daß die in der Kulturflüssigkeit erzielbare Zelldichte nicht durch die besagte Sauerstofflimitierung bei der Passage der Zellen durch das Membranmodul begrenzt wird. Zwar war die Verwendung von Dialysemembranen zur Entfernung von toxischen Stoffwechselprodukten, wie auch eingangs bereits ausgeführt, bekannt, jedoch war nicht bekannt, daß bei Verwendung von entsprechenden Membranen gleichzeitig die vorstehend geschilderte Sauerstoffversorgung gewährleistet werden kann. Es besteht somit ein Zusammenhang zwischen der erfindungsgemäßen Vorrichtung sowie der erfindungsgemäßen Verwendung dergestalt, daß das zu lösende Problem darin bestand, eine Sauerstofflimitierung der Zellen während deren Passage durch das Membranmodul zu vermeiden. Dies wird im Falle der erfindungsgemäßen Vorrichtung dadurch erreicht, daß bevorzugt in den Innenraum der membranförmigen Membranen Sauerstoff eingeblasen wird und im Falle der erfindungsgemäßen Verwendung der kommerziell erhältlichen Plattenmodule solche ausgewählt werden mit einem ausreichend großen Flächen-Volumen-Verhältnis sowie ausreichend hohem Sauerstoffpermeabilitätskoeffizienten, so daß die im Behältnis für die Dialyseflüssigkeit 1 mit Sauerstoff angereicherte Dialyseflüssigkeit diesen über die Dialysemembran an die durch das Membranmodul passagierenden Zellen abgeben kann. In beiden Fällen wird das Problem der Sauerstofflimitierung der das Membranmodul passierenden Zellen gelöst.

Ein vorstehend beschriebenes und in Fig. 4 schematische dargestelltes Membranmodul kann in dem gattungsgemäßen Reaktionssystem verwendet werden, wie dies beispielsweise in Fig. 2 dargestellt ist.

Die in der vorstehenden Beschreibung, den Ansprüchen sowie den Zeichnungen offenbarten Merkmale der Erfindung können sowohl einzeln als auch in beliebigen Kombinationen für die Verwirklichung der Erfindung in ihren verschiedenen Ausführungsformen wesentlich sein.

Bezugszeichenliste

| | |
|---|---|
| 1 | Behälter für Dialyseflüssigkeit |
| 2 | Kulturraum |
| 3 | Dialysemembran |
| 4 | Rührwerk (im Kulturraum) |
| 5 | Gaszufuhrleitung |
| 6 | Abluftleitung |
| 7 | Gasbläschen |
| 8 | Membranmodul |
| 9 | Pumpe (für Dialyseflüssigkeit) |
| 10 | Pumpe (für Kulturflüssigkeit) |
| 11 | Leitung (für Dialyseflüssigkeit) |
| 12 | Leitung (für Dialyseflüssigkeit) |
| 13 | Leitung (für Kultursuspension) |
| 14 | Ableitung (für Kultursuspension) |
| 15 | Zufuhrleitung (für Röhrchen 16) |
| 16 | Röhrchen |
| 17 | Kulturflüssigkeit |
| 18 | Dialyseflüssigkeit |
| 19 | Stützstrukturen |
| 20 | Leitung (zum Membranmodul) |
| 21 | Gaszufuhrleitung |
| 22 | Abluftleitung |
| 23 | Rührwerk (im Behälter für Dialyseflüssigkeit) |

**Patentansprüche**

1. Verfahren zur Kultivierung von Zellen unter Verwendung eines Reaktionssystems, wobei das Reaktionssystem einen Behälter für Dialyseflüssigkeit, einen Raum für die Kultivierung von Zellen sowie ein beide Räume miteinander verbindendes Membranmodul umfaßt, wobei das Membranmodul mindestens zwei durch eine Membran getrennte Räume aufweist, deren einer von einer Dialyseflüssigkeit und deren anderer von Zellen enthaltender Kulturflüssigkeit durchströmt wird, und ein erstes Gas in die Kulturflüssigkeit in dem Raum für die Kultivierung der Zellen eingetragen wird, **dadurch gekennzeichnet, daß** ein zweites Gas in die Kulturflüssigkeit im Membranmodul eingetragen wird und die Membran funktional eine Dialysemembran ist.

2. Verfahren nach Anspruch 1, **dadurch gekennzeichnet, daß** eine direkte Begasung der im Membranmodul befindlichen Kulturflüssigkeit erfolgt, indem das zweite Gas direkt in die Kulturflüssigkeit im Membranmodul eingetragen wird.

3. Verfahren nach Anspruch 1, **dadurch gekennzeichnet, daß** eine indirekte Begasung der im Membranmodul befindlichen Kulturflüssigkeit erfolgt, indem das zweite Gas in die Dialyseflüssigkeit in dem Behälter für die Dialyseflüssigkeit eingetragen wird und von dort über die Membran des Membranmoduls in die im Membranmodul befindliche Kulturflüssigkeit gelangt.

4. Verfahren nach Anspruch 2 oder 3, **dadurch gekennzeichnet, daß** neben der direkten Begasung gleichzeitig die indirekte Begasung erfolgt.

5. Verfahren nach einem der Ansprüche 1 bis 4, **dadurch gekennzeichnet, daß** das Membranmodul mindestens ein Gaszufuhrmittel umfaßt und das Gaszufuhrmittel das zweite Gas zu mindestens einem der durch die Membran getrennten, eine Flüssigkeit führenden Räume zuführt.

6. Verfahren nach Anspruch 5, **dadurch gekennzeichnet, daß** das Gaszufuhrmittel eine Austrittsöffnung aufweist, die sich insbesondere in dem die Kulturflüssigkeit führenden Raum des Membranmoduls befindet.

7. Verfahren nach Anspruch 5 oder 6, **dadurch gekennzeichnet, daß** das Gaszufuhrmittel ein Röhrchen ist.

8. Verfahren nach einem der Ansprüche 5 bis 7, **dadurch gekennzeichnet, daß** das Gaszufuhrmittel als Düse ausgebildet ist.

9. Verfahren nach einem der Ansprüche 1 bis 8, **dadurch gekennzeichnet, daß** die Membran des Membranmoduls aus einem Material besteht, das ausgewählt ist der Gruppe, die regenerierte Cellulose, Polyamid, Polypropylen und Polysulfon umfaßt.

10. Verfahren nach Anspruch 1 bis 4 und 9, **dadurch gekennzeichnet, daß** das Membranmodul ein Plattenmodul ist.

11. Verfahren nach Anspruch 10, **dadurch gekennzeichnet, daß** die Membran des Membranmoduls eine Dialysemembran ist.

12. Verfahren nach Anspruch 11, **dadurch gekennzeichnet, daß** das Material der Dialysemembran Cuprophan ist

13. Verfahren nach einem der Ansprüche 1 bis 12, **dadurch gekennzeichnet, daß** das Membranmodul bedingt durch sein Flächen-Volumen-Verhältnis und seinen Permeabilitätskoeffzienten für das Gas einen für die Zellen ausreichenden Gasaustausch vorsieht.

14. Verfahren nach Anspruch 13, **dadurch gekennzeichnet, daß** das Flächen-Volumen-Verhältnis mindestens etwa 5 $m^2$ pro Liter, insbesondere mindestens 10 $m^2$ pro Liter beträgt.

15. Verfahren nach Anspruch 14 **dadurch gekennzeichnet, daß** das Flächen-Volumen-Verhältnis etwa 13 $m^2$ pro Liter beträgt.

16. Verfahren nach einem der Ansprüche 13 bis 15, **dadurch gekennzeichnet, daß** der Sauerstoff-Permeabilitätskoeffizient etwa 0,066 cm pro Minute oder mehr beträgt.

17. Verfahren nach einem der Ansprüche 1 bis 16, **dadurch gekennzeichnet, daß** der Behälter für Dialyseflüssigkeit ein Mittel zum Zuführen von Gas und/oder ein Mittel zum Abführen von Gas aufweist.

18. Verfahren nach einem der Ansprüche 1 bis 17, **dadurch gekennzeichnet, daß** das Membranmodul, der Raum für die Kultivierung der Zellen und/oder der Behälter für Dialyseflüssigkeit einen erhöhten Druck aufweist.

**19.** Verfahren nach einem der Ansprüche 1 bis 18, **dadurch gekennzeichnet, daß** das erste und zweite Gas einzeln und unabhängig voneinander ausgewählt sind aus der Gruppe, die Luft, Sauerstoff, Stickstoff, Kohlendioxid und Mischungen davon umfaßt.

**20.** Verfahren nach Anspruch 19, **dadurch gekennzeichnet, daß** das zweite Gas Sauerstoff ist.

**21.** Verfahren nach Anspruch 19, **dadurch gekennzeichnet, daß** das zweite Gas Kohlendioxid ist.

**22.** Verfahren nach einem der Ansprüche 1 bis 21, **dadurch gekennzeichnet, daß** die Zellen ausgewählt sind aus der Gruppe, die mikrobielle Zellen, Pilzzellen, tierische Zellen und pflanzliche Zellen umfaßt.

**23.** Verfahren nach Anspruch 22, **dadurch gekennzeichnet, daß** die Zellen Zellen von Escherichia coli sind.

**24.** Membranmodul welches mindestens zwei durch eine Dialysemembran getrennte Räume umfaßt und jeder Raum mit einer Flüssigkeit durchströmt wird, wobei die Flüssigkeit in einem Raum Dialyseflüssigkeit und in dem anderen Raum Kulturflüssigkeit ist, **dadurch gekennzeichnet, daß** das Membranmodul mindestens ein Gaszufuhrmittel umfaßt und das Gaszufuhrmittel in mindestens einem der durch die Dialysemembran getrennten Räume eine Austrittsöffnung aufweist.

**25.** Membranmodul nach Anspruch 24, **dadurch gekennzeichnet, daß** die Membran röhrenförmig ausgebildet ist und durch ihr Volumen einen der beiden Räume ausbildet.

**26.** Membranmodul nach Anspruch 25, **dadurch gekennzeichnet, daß** der Durchmesser des durch die röhrenförmige Membran ausgebildeten Raumes etwa 3 - 10 mm, bevorzugt 6 - 8 mm, beträgt.

**27.** Membranmodul nach Anspruch 25 oder 26, **dadurch gekennzeichnet, daß** das Membranmodul mehrere durch eine röhrenförmige Membran ausgebildete Räume umfaßt.

**28.** Membranmodul nach Anspruch 27, **dadurch gekennzeichnet, daß** sich in mindestens einem der durch die röhrenförmige Membran ausgebildeten Räume eine Austrittsöffnung des Gaszufuhrmittels befindet.

**29.** Membranmodul nach einem der Ansprüche 24 bis 28, **dadurch gekennzeichnet, daß** sich die Austrittsöffnung des Gaszufuhrmittels in einem Raum außerhalb des/der durch die röhrenförmige Membran ausgebildeten Raumes/Räume befindet.

**30.** Membranmodul nach einem Ansprüche 24 bis 29, **dadurch gekennzeichnet, daß** das Gaszufuhrmittel ein Röhrchen ist.

**31.** Membranmodul nach Anspruch 30, **dadurch gekennzeichnet, daß** das Röhrchen konzentrisch in dem Raum angeordnet ist.

**32.** Membranmodul nach Anspruch 30 oder 31, **dadurch gekennzeichnet, daß** das Röhrchen einen Innendurchmesser von 0,2 mm bis 3 mm aufweist.

**33.** Membranmodul nach einem der Ansprüche 24 bis 32, **dadurch gekennzeichnet, daß** die Austrittslösung des Gaszufuhrmittels als Düse ausgebildet ist.

**34.** Verwendung des Membranmoduls nach einem der Ansprüche 24 bis 33 als Membranmodul in einem Verfahren nach einem der Ansprüche 1 bis 23.

**35.** Reaktionssystem zur Kultivierung von E. coli Zellen umfassend einen Behälter für Dialyseflüssigkeit, einen Raum für die Kultivierung von Zellen und mindestens ein dazwischen geschaltetes Membranmodul, **dadurch gekennzeichnet, daß** das Membranmodul ein solches nach einem der Ansprüche 24 bis 33 ist.

**36.** Reaktionssystem nach Anspruch 35, **dadurch gekennzeichnet, daß** der Behälter für Dialyseflüssigkeit mindestens eine Begasungseinrichtung enthält.

**Claims**

1. A method for culturing cells using a reaction system, in which the reaction system comprises a container for dialysis fluid, a space for culturing cells and a membrane module which connects both spaces with one another and which has at least two spaces which are separated by a membrane and through one of which a dialysis fluid flows and through the second of which culture fluid containing cells flows, and a first gas is introduced into the culture fluid in the space for culturing the cells, **characterized in that** a second gas is introduced into the culture fluid in the membrane module and the membrane is functionally a dialysis membrane.

2. The method as claimed in claim 1, **characterized in that** gas is passed directly into the culture fluid present in the membrane module by introducing the second gas directly into the culture fluid in the membrane module.

3. The method as claimed in claim 1, **characterized in that** gas is passed indirectly into the culture fluid present in the membrane module by introducing the second gas into the dialysis fluid in the container for the dialysis fluid and the gas reaching from there the culture fluid present in the membrane module via the membrane of the membrane module.

4. The method as claimed in claim 2 or 3, **characterized in that** gas is introduced both directly and indirectly at the same time.

5. The method as claimed in any of claims 1 to 4, **characterized in that** the membrane module comprises at least one gas supplying means and the gas supplying means supplies at least one of the spaces separated by the membrane and carrying a liquid with the second gas.

6. The method as claimed in claim 5, **characterized in that** the gas supplying means has an outlet which is located in particular in the membrane module space carrying the culture fluid.

7. The method as claimed in claim 5 or 6, **characterized in that** the gas supplying means is a tube.

8. The method as claimed in any of claims 5 to 7, **characterized in that** the gas supplying means has the shape of a nozzle.

9. The method as claimed in any of claims 1 to 8, **characterized in that** the membrane of the membrane module is composed of a material which is selected from the group comprising regenerated cellulose, polyamide, polypropylene and polysulfone.

10. The method as claimed in claims 1 to 4 and 9, **characterized in that** the membrane module is a plate module.

11. The method as claimed in claim 10, **characterized in that** the membrane of the membrane module is a dialysis membrane.

12. The method as claimed in claim 11, **characterized in that** the dialysis membrane material is Cuprophan.

13. The method as claimed in any of claims 1 to 12, **characterized in that** the membrane module provides for a gas exchange sufficient for the cells, due to its area/volume ratio and its gas permeability coefficient.

14. The method as claimed in claim 13, **characterized in that** the area/volume ratio is at least about 5 $m^2$ per liter, in particular at least 10 $m^2$ per liter.

15. The method as claimed in claim 14, **characterized in that** the area/volume ratio is about 13 $m^2$ per liter.

16. The method as claimed in any of claims 13 to 15, **characterized in that** the oxygen permeability coefficient is about 0.066 cm per minute or higher.

17. The method as claimed in any of claims 1 to 16, **characterized in that** the container for dialysis fluid has a means for supplying gas and/or a means for removing gas.

18. The method as claimed in any of claims 1 to 17, **characterized in that** the membrane module, the space for culturing the cells and/or the container for dialysis fluid have increased pressure.

19. The method as claimed in any of claims 1 to 18, **characterized in that** the first and the second gas are individually and independently of one another selected from the group comprising air, oxygen, nitrogen, carbon dioxide and mixtures thereof.

20. The method as claimed in claim 19, **characterized in that** the second gas is oxygen.

21. The method as claimed in claim 19, **characterized in that** the second gas is carbon dioxide.

22. The method as claimed in any of claims 1 to 21, **characterized in that** the cells are selected from the group comprising microbial cells, fungal cells, animal cells and plant cells.

23. The method as claimed in claim 22, **characterized in that** the cells are Escherichia coli cells.

24. A membrane module which comprises at least two spaces separated by a dialysis membrane and through each space a liquid flows, the liquid in one space being dialysis fluid and in the other space culture fluid, **characterized in that** the membrane module comprises at least one gas supplying means and the gas supplying means in at least one of the spaces separated by the dialysis membrane has an outlet.

25. The membrane module as claimed in claim 24, **characterized in that** the membrane has the shape of a tube and its volume forms one of the two spaces.

26. The membrane module as claimed in claim 25, **characterized in that** the diameter of the space formed by the tube-shaped membrane is about 3-10 mm, preferably 6-8 mm.

27. The membrane module as claimed in claim 25 or 26, **characterized in that** the membrane module comprises a plurality of spaces formed by a tube-shaped membrane.

28. The membrane module as claimed in claim 27, **characterized in that** a gas supplying means outlet is located in at least one of the spaces formed by the tube-shaped membrane.

29. The membrane module as claimed in any of claims 24 to 28, **characterized in that** the gas supplying means outlet is located in a space outside the space(s) formed by the tube-shaped membrane.

30. The membrane module as claimed in any of claims 24 to 29, **characterized in that** the gas supplying means is a tube.

31. The membrane module as claimed in claim 30, **characterized in that** the tube is arranged in the space in a concentric manner.

32. The membrane module as claimed in claim 30 or 31, **characterized in that** the internal diameter of the tube is from 0.2 mm to 3 mm.

33. The membrane module as claimed in any of claims 24 to 32, **characterized in that** the gas supplying means outlet has the shape of a nozzle.

34. The use of the membrane module as claimed in any of claims 24 to 33 as membrane module in a method as claimed in any of claims 1 to 23.

35. A reaction system for culturing E. coli cells comprising a container for dialysis fluid, a space for culturing cells and at least one membrane module inserted in between, **characterized in that** the membrane module is that as claimed in any of claims 24 to 33.

36. The reaction system as claimed in claim 35, **characterized in that** the container for dialysis fluid contains at least one gas-introducing device.

**Revendications**

1. Procédé pour la culture de cellules au moyen d'un système de réaction, le système de réaction comprenant un récipient pour le liquide de dialyse, un espace pour la culture de cellules ainsi qu'un module membranaire reliant l'un à l'autre les deux espaces, le module membranaire comportant au moins deux espaces séparés par une membrane, dont un est traversé par un liquide de dialyse et dont l'autre est traversé par le liquide de culture contenant des cellules, et un premier gaz étant introduit dans le liquide de culture dans l'espace pour la culture des cellules, **caractérisé en ce qu'**un second gaz est introduit dans le liquide de culture dans le module membranaire et la membrane est quant à sa fonction une membrane de dialyse.

2. Procédé selon la revendication 1, **caractérisé en ce qu'**un apport direct de gaz au liquide de culture présent dans le module membranaire s'effectue par introduction directe du second gaz dans le liquide de culture dans le module membranaire.

3. Procédé selon la revendication 1, **caractérisé en ce que** qu'un apport indirect de gaz au liquide de culture présent dans le module membranaire s'effectue par introduction du second gaz dans le liquide de dialyse dans le récipient pour le liquide de dialyse et, de là, en passant à travers la membrane du module membranaire, aboutit dans le liquide de culture se trouvant dans le module membranaire.

4. Procédé selon la revendication 2 ou 3, **caractérisé en ce que**, outre l'apport direct de gaz, l'apport indirect de gaz s'effectue en même temps.

5. Procédé selon l'une quelconque des revendications 1 à 4, **caractérisé en ce que** le module membranaire comprend au moins un moyen d'arrivée de gaz et le moyen d'arrivée de gaz amène le second gaz à au moins l'un des espaces conduisant un liquide, séparés par la membrane.

6. Procédé selon la revendication 5, **caractérisé en ce que** le moyen d'arrivée de gaz présente un orifice de sortie qui se trouve en particulier dans l'espace du module membranaire qui conduit le liquide de culture.

7. Procédé selon la revendication 5 ou 6, **caractérisé en ce que** le moyen d'arrivée de gaz est un petit tube.

8. Procédé selon l'une quelconque des revendications 5 à 7, **caractérisé en ce que** le moyen d'arrivée de gaz est en forme de buse.

9. Procédé selon l'une quelconque des revendications 1 à 8, **caractérisé en ce que** la membrane du module membranaire est constituée d'un matériau qui est choisi dans le groupe qui comprend la cellulose régénérée, le polyamide, le polypropylène et la polysulfone.

10. Procédé selon l'une quelconque des revendications 1 à 4 et 9, **caractérisé en ce que** le module membranaire est un module à plaques.

11. Procédé selon la revendication 10, **caractérisé en ce que** la membrane du module membranaire est une membrane de dialyse.

12. Procédé selon la revendication 11, **caractérisé en ce que** le matériau de la membrane de dialyse est le cuprophan.

13. Procédé selon l'une quelconque des revendications 1 à 12, **caractérisé en ce que** le module membranaire procure un échange de gaz suffisant pour les cellules, en raison de son rapport surface-volume et de ses coefficients de perméabilité.

14. Procédé selon la revendication 13, **caractérisé en ce que** le rapport surface-volume est d'au moins environ 5 $m^2$ par litre, en particulier d'au moins 10 $m^2$ par litre.

15. Procédé selon la revendication 14, **caractérisé en ce que** le rapport surface-volume est d'environ 13 $m^2$ par litre.

16. Procédé selon l'une quelconque des revendications 13 à 15, **caractérisé en ce que** le coefficient de perméabilité à l'oxygène est d'environ 0,066 cm par minute ou plus.

**17.** Procédé selon l'une quelconque des revendications 1 à 16, **caractérisé en ce que** le récipient pour liquide de dialyse comporte un moyen pour l'arrivée de gaz et/ou un moyen pour l'évacuation de gaz.

**18.** Procédé selon l'une quelconque des revendications 1 à 17, **caractérisé en ce que** l'espace pour la culture des cellules et/ou le récipient pour liquide de dialyse présente une pression accrue.

**19.** Procédé selon l'une quelconque des revendications 1 à 18, **caractérisé en ce que** le premier et le second gaz sont choisis, individuellement et indépendamment l'un de l'autre, dans le groupe qui comprend l'air, l'oxygène, l'azote, le dioxyde de carbone et des mélanges de ceux-ci.

**20.** Procédé selon la revendication 19, **caractérisé en ce que** le second gaz est l'oxygène.

**21.** Procédé selon la revendication 19, **caractérisé en ce que** le second gaz est le dioxyde de carbone.

**22.** Procédé selon l'une quelconque des revendications 1 à 21, **caractérisé en ce que** les cellules sont choisies dans le groupe qui comprend des cellules microbiennes, des cellules de champignons, des cellules animales et des cellules végétales.

**23.** Procédé selon la revendication 22, **caractérisé en ce que** les cellules sont des cellules d'*Escherichia coli.*

**24.** Module membranaire qui comprend au moins deux espaces séparés par une membrane de dialyse et chaque espace est traversé par un liquide, le liquide étant dans un espace le liquide de dialyse et dans l'autre espace le liquide de culture, en ce que le module membranaire comprend au moins un moyen d'arrivée de gaz et le moyen d'arrivée de gaz présente un orifice de sortie dans au moins l'un des espaces séparés par la membrane de dialyse.

**25.** Module membranaire selon la revendication 24, **caractérisé en ce que** la membrane a une forme tubulaire et constitue par son volume l'un des deux espaces.

**26.** Module membranaire selon la revendication 25, **caractérisé en ce que** le diamètre de l'espace formé par la membrane tubulaire est d'environ 3-10 mm, de préférence 6-8 mm.

**27.** Module membranaire selon la revendication 25 ou 26, **caractérisé en ce que** le module membranaire comprend plusieurs espaces formés par une membrane tubulaire.

**28.** Module membranaire selon la revendication 27, **caractérisé en ce que** dans au moins l'un des espaces formés par la membrane tubulaire se trouve un orifice de sortie du moyen d'arrivée de gaz.

**29.** Module membranaire selon l'une quelconque des revendications 24 à 28, **caractérisé en ce que** l'orifice de sortie du moyen d'arrivée de gaz se trouve dans un espace à l'extérieur de l'espace/des espaces formé(s) par la membrane tubulaire.

**30.** Module membranaire selon l'une quelconque des revendications 24 à 29, **caractérisé en ce que** le moyen d'arrivée de gaz est un petit tube.

**31.** Module membranaire selon la revendication 30, **caractérisé en ce que** le petit tube est disposé concentriquement dans l'espace ;

**32.** Module membranaire selon la revendication 30 ou 31, **caractérisé en ce que** le petit tube présente un diamètre interne de 0,2 mm à 3 mm.

**33.** Module membranaire selon l'une quelconque des revendications 24 à 32, **caractérisé en ce que** l'orifice de sortie du moyen d'arrivée de gaz est sous forme de buse.

**34.** Utilisation du module membranaire selon l'une quelconque des revendications 24 à 33, en tant que module membranaire dans un procédé selon l'une quelconque des revendications 1 à 23.

**35.** Système de réaction pour la culture de cellules de *E. coli*, comprenant un récipient pour liquide de dialyse, un espace pour la culture de cellules et au moins un module membranaire intercalé entre ceux-ci, **caractérisé en ce que** le module membranaire est un module membranaire selon l'une quelconque des revendications 24 à 33.

**36.** Système de réaction selon la revendication 35, **caractérisé en ce que** le récipient pour liquide de dialyse contient au moins un dispositif d'apport de gaz.

# Fig. 1

Fig. 2

Fig. 3

19  18  17  3

Fig. 4